(19) Europäisches Patentamt
European Patent Office
Office européen des brevets

(11) EP 0 641 317 B1

# (12) EUROPEAN PATENT SPECIFICATION

(45) Date of publication and mention of the grant of the patent: **18.09.1996 Bulletin 1996/38**

(21) Application number: **92916724.5**

(22) Date of filing: **29.07.1992**

(51) Int Cl.[6]: **C07C 317/24**, A01N 41/10

(86) International application number: **PCT/US92/06276**

(87) International publication number: **WO 93/03009 (18.02.1993 Gazette 1993/05)**

(54) **2-(2-CHLORO-3-ETHOXY-4-ETHYLSULFONYL BENZOYL)-5-METHYL-1,3-CYCLOHEXANEDIONE AS HERBICIDE**

2-(2-CHLORO-3-ETHOXY-4-ETHYLSULFONYL BENZOYL)-5-METHYL-1,3-CYCLOHEXANDION ALS HERBIZID

2-(2-CHLORO-3-ETHOXY-4-ETHYLESULFONYLE BENZOYLE)-5-METHYLE-1,3-CYCLOHEXANEDIONE UTILISE COMME HERBICIDE

(84) Designated Contracting States: **AT BE DE DK FR GB IT LU MC NL SE**

(30) Priority: **01.08.1991 US 739281**

(43) Date of publication of application: **08.03.1995 Bulletin 1995/10**

(73) Proprietor: **ZENECA INC.**
**Wilmington, Delaware 19897 (US)**

(72) Inventor: **LEE, David, L.**
**Pleasant Hill, CA 94523 (US)**

(74) Representative: **Ricks, Michael James et al**
**Intellectual Property Department**
**ZENECA Agrochemicals**
**Jealotts Hill Research Station**
**P.O. Box 3538**
**Bracknell Berkshire RG12 6YA (GB)**

(56) References cited:
**WO-A-90/05712** **US-A- 4 780 127**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

U.S. Patent 4,780,127 relates to certain 2-(substituted benzoyl)-1,3-cyclohexanediones and their use as herbicides. The compound of this invention is generically taught by this patent.

Description of the Invention

This invention relates to 2-(2-chloro-3-ethoxy-4-ethylsulfonyl benzoyl)-5-methyl-1,3-cyclohexanedione, its herbicidal use and to herbicidal compositions containing the compound.

The compound of this invention has the structural formula

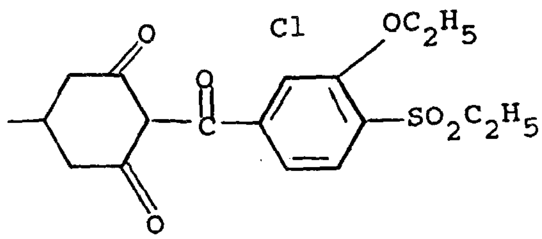

Salts of the above-described compound are also the subject of the instant invention.

The compound of this invention can have the following four structural formulae because of tautomerism:

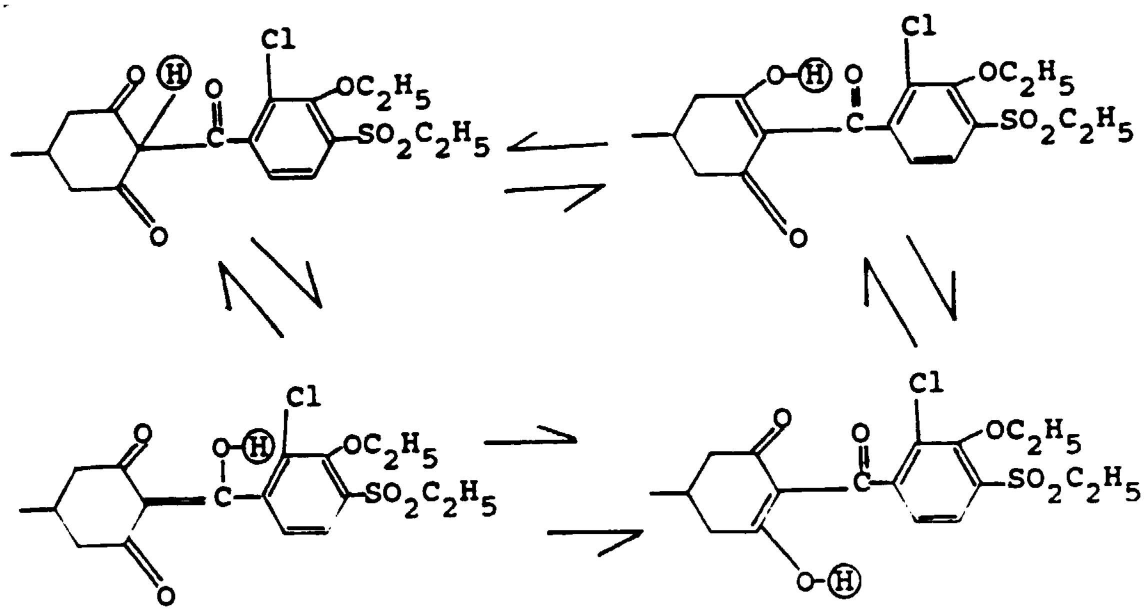

The circled proton on each of the four tautomers is reasonably labile. These protons are acidic and can be removed by any base to give a salt having an anion of the following four resonance forms:

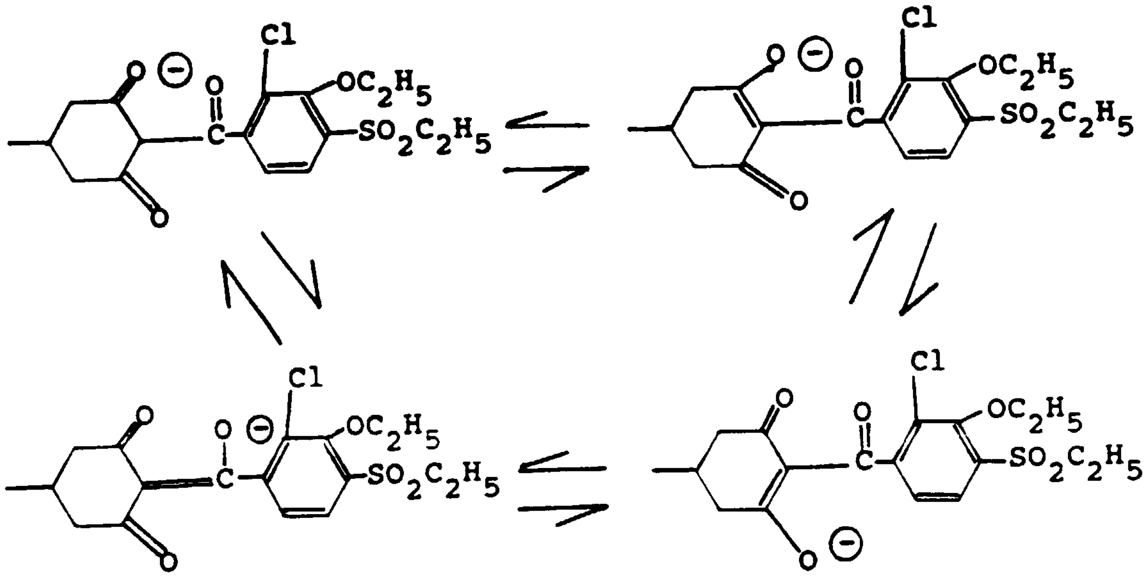

Examples of cations of these bases are inorganic cations such as alkali metals e.g. lithium, sodium, and potassium or organic cations such as substituted ammonium, sulfonium or phosphonium wherein the substituent is an aliphatic or aromatic group.

The compound of this invention and its salts have unexpected exceptional herbicidal activity against certain plant species and have unexpected exceptional crop selectivity.

The compound of the present invention can be prepared by the following two-step general method.

The process proceeds via the production of an enol ester intermediate as shown in reaction (1). The final product is obtained by rearrangement of the enol ester as shown in reaction (2). The two reactions may be conducted as separate steps by isolation and recovery of the enol ester using conventional techniques prior to conducting step (2), or by addition of a cyanide source to the reaction medium after the formation of the enol ester, or in one step by inclusion of the cyanide source at the start of reaction (1).

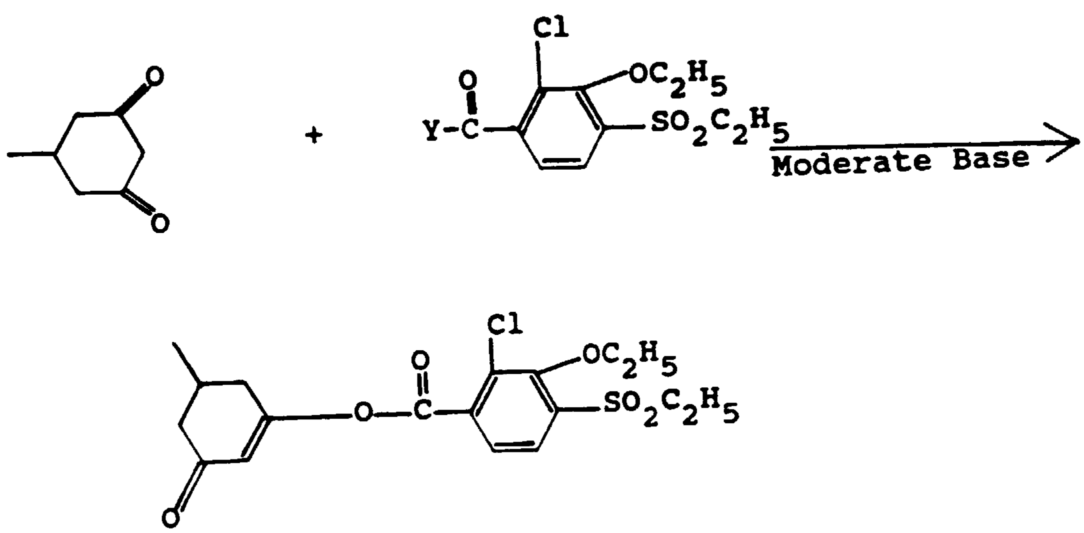


wherein Y is halogen, preferably chlorine, $C_1$-$C_4$ alkyl-C(O)-O-, $C_1$-$C_4$ alkoxy-C(O)-O or

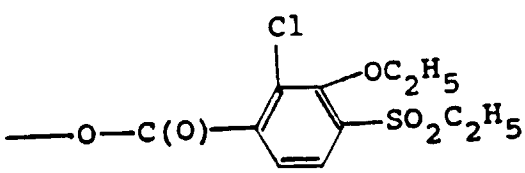

wherein the moderate base is as defined, preferably tri-$C_1$-$C_6$ alkylamine, pyridine, alkali metal carbonate or alkali metal phosphate.

Generally, in step (1) mole amounts of the dione and substituted benzoyl reactant are used, along with a mole amount or excess of the base. The two reactants are combined in an organic solvent such as methylene chloride, toluene, ethyl acetate or dimethylformamide. The base or benzoyl reactant preferably are added to the reaction mixture with cooling. The mixture is stirred at 0°C-50°C until the reaction is substantially complete.

The reaction product is worked up by conventional techniques.

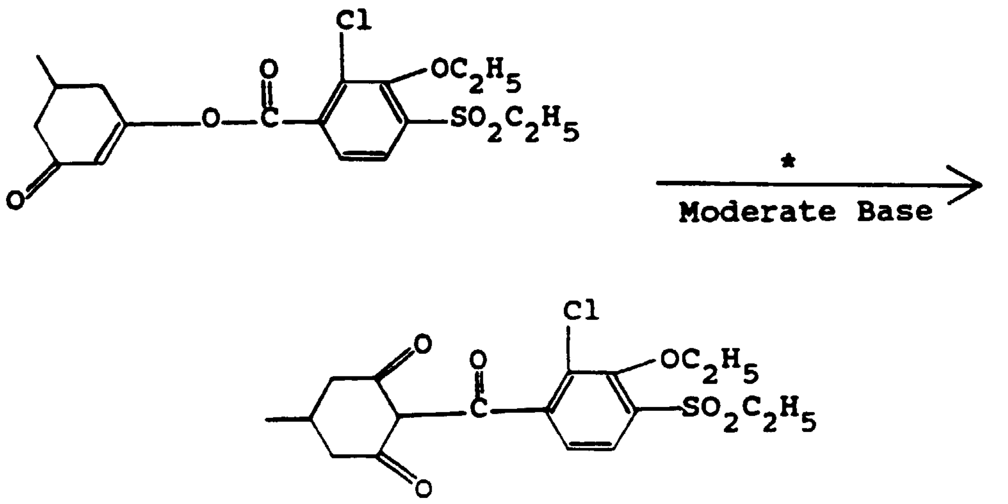


* = Cyanide source. Moderate base = as defined herein.

Generally, in step (2) a mole of the enol ester intermediate is reacted with 1 to 4 moles of the base, preferably about 2 moles of moderate base and from 0.01 mole to about 0.5 mole or higher, preferably around 0.1 mole of the cyanide source (e.g., potassium cyanide or acetone cyanohydrin). The mixture is stirred in a reaction pot until the rearrangement is substantially complete at a temperature below 80°C, preferably about 20°C to about 40°C, and the desired product is recovered by conventional techniques.

The term "cyanide source" refers to a substance or substances which under the rearrangement conditions consists of or generates hydrogen cyanide and/or cyanide anion.

The process is conducted in the presence of a catalytic amount of a source of cyanide anion and/or hydrogen cyanide, together with a molar excess, with respect to the enol ester, of a moderate base.

Preferred cyanide sources are alkali metal cyanides such as sodium and potassium cyanide; cyanohydrins of methyl alkyl ketones having from 1-4 carbon atoms in the alkyl groups, such as acetone or methyl isobutyl ketone cyanohydrins; cyanohydrins of benzaldehyde or of $C_2$-$C_5$ aliphatic aldehydes such as acetaldehyde, propionaldehyde, etc., cyanohydrins; zinc cyanide; tri(lower alkyl) silyl cyanides, notably trimethyl silyl cyanide; and hydrogen cyanide itself. Hydrogen cyanide is considered most advantageous as it produces relatively rapid reaction and is inexpensive. Among cyanohydrins the preferred cyanide source is acetone cyanohydrin.

The cyanide source is used in an amount up to about 50 mole percent based on the enol ester. It may be used in as little as about 1 mole percent to produce an acceptable rate of reaction at about 40°C on a small scale. Larger scale reactions give more reproducible results with slightly higher catalyst levels of about 2 mole percent. Generally about 1-10 mole percent of the cyanide source is preferred.

The process is conducted with a molar excess, with respect to the enol ester, of a moderate base. By the term "moderate base" is meant a substance which acts as a base yet whose strength or activity as a base lies between that of strong bases such as hydroxides (which could cause hydrolysis of the enol ester) and that of weak bases such as bicarbonates (which would not function effectively). Moderate bases suitable for use in this embodiment include both organic bases such as tertiary amines and inorganic bases such as alkali metal carbonates and phosphates. Suitable tertiary amines include trialkylamines such as triethylamine, trialkanolamines such as triethanolamine, and pyridine. Suitable inorganic bases include potassium carbonate and trisodium phosphate.

The base is used in an amount of from about 1 to about 4 moles per mole of enol ester, preferably about 2 moles per mole.

When the cyanide source is an alkali metal cyanide, particularly potassium cyanide, a phase transfer catalyst may be included in the reaction. Particularly suitable phase transfer catalysts are the Crown ethers.

A number of different solvents may be usable in this process, depending on the nature acid chloride or the acylated product. A preferred solvent for this reaction is 1,2-dichloroethane. Other solvents which may be employed, depending

on the reactants or products include toluene, acetonitrile, methylene chloride, ethyl acetate, dimethylformamide, and methyl isobutyl ketone (MIBK).

In general, depending on the nature of the reactants and the cyanide source, the rearrangement may be conducted at temperatures up to about 50°C.

The above-described substituted benzoyl chloride can be prepared from the corresponding substituted benzoic acid according to the teaching of Reagents for Organic Synthesis, Vol I, L. G. Fieser and M. Fieser, pp. 767-769 (1967).

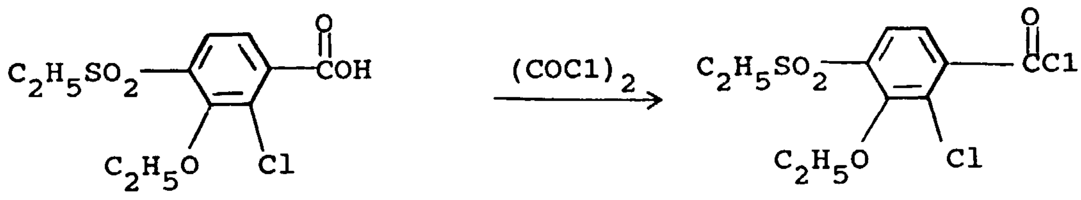


The compound of this invention can be prepared as indicated in the following example.

EXAMPLE

2-(2-chloro-3-ethoxy-4-ethylsulfonyl benzoyl)-5-methyl-1,3-cyclohexanedione

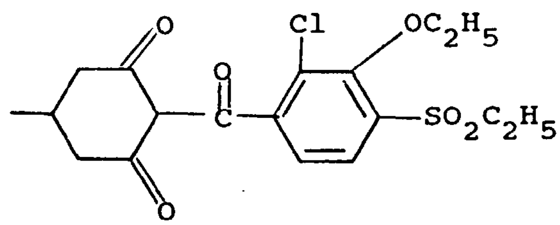

5-Methyl-1,3-cyclohexanedione (439.0 grams, 3.416 mole), triethylamine (1.43 liters, 10.248 mole) and 1 liter methylene chloride were added to a 12 liter, 4 neck, round bottom flask fitted with a thermometer, condenser, addition funnel, mechanical stirrer, catch basin and nitrogen sweep. The reaction mixture had a slightly cloudy pink color. The reaction mixture was cooled with a water bath to 20°C and 2-chloro-3-ethoxy-4-ethylsulfonylbenzoyl chloride (1,108.66 grams, 3.416 moles) dissolved in 1 liter methylene chloride were added to the reaction mixture over 15 minutes. An exothermic reaction raised the temperature of the mixture to 38°C. The reaction mixture had a reddish brown color with solids. After 5 minutes, trimethylsilyl cyanide (35 mil, 0.262 mole) was added to the reaction mixture at 35°C. The reaction mixture had a creamy brown color with solids. The reaction mixture was warmed to 40°C. An exothermic reaction raised the temperature to 46°C. The reaction mixture was cooled to 40°C then maintained at 35 - 40°C. After 1 hour reaction time at 40°C, high performance liquid chromatography showed no enolester and 86.9A% product. After 2 hours at 35°C with a creamy reddish brown color, high performance liquid chromatography showed no enolester and 87.3A% product. After 2-1/2 hours, the reaction mixture was cooled to 20°C with a water bath. The reaction mixture was washed one time with 3 liters water, pH 10, one time with 4 liters hydrochloric acid (3.0 molar), the methylene chloride was dried over magnesium sulfate, filtered and stripped on a water aspirator at a temperature ranging from room temperature to 40°C for 2 hours until foamy. 1,549 grams clear dark brown oil was obtained. The oil was diluted in 1 liter ethyl ether and poured into a 4 liter beaker. The solution was stirred until the beginning of crystallization, then quickly and additional 1.2 liters of ethyl ether was added with stirring and cooled to 5°C for 1/2 hour with an ice bath. The solids were filtered, rinsed with 1.5 liters cold ethyl ether, dried under suction for 20 minutes, trayed and dried in an oven at 55°C for 2-1/2 hours. 1,065.2 grams of the desired product, beige powdery solids, was obtained. 77.8% yield. High performance liquid chromatography showed that 100% of the desired product was obtained, m.p. 118.5-120.5°C.

The nuclear magnetic resonance, infrared and mass spectroscopy data for the desired compound is as follows:

H-NMR($CDCl_3$): δ 1.13 (d, 3H), 1.25(t, 3H), 1.47(t, 3H), 2.16(m, 1H), 2.32 (m, 1H), 2.53 (m, 2H), 2.83(dd, 1H), 3.44(g, 2H), 4.29(q, 2H), 7.06(d, 1H), 7.91(d, 1H), 16.71(br.s,1H)

IR (film): wave number ($cm^{-1}$) 3095, 2973, 2961, 2916, 1677, 1551, 1452, 1422, 1407, 1382, 1312, 1140, 1016, 875, 822, 804, 768, 719

MS (m/z): 55(25), 69(100), 77(37), 153(10), 228(10), 244(7), 337(4), 365(10, $M^+$-Cl) , no $M^+$

Herbicidal Screening Tests

As previously mentioned, the herein-described compounds produced in the above-described manner are phytotoxic compounds which are useful and valuable in controlling various plant species. Selected compounds of this invention were tested as herbicides in the following manner.

Preemergence Multi-weed Herbicide Test: On the day preceding treatment, seeds of eight different weed species are planted in loamy sand soil in individual rows using one species per row across the width of the flat. The weeds used are giant foxtail (GFT) (Setenia faberi), green foxtail (FT) (Setaria viridis), watergrass (WG) (Echinochloa crusgalli), broadleaf signalgrass (BSG) (Brachiaria platyphylla), annual morning glory (AMG) (Ipomoea lacunosa), velvetleaf (VL) (Abutilon theophrasti), cocklebur (CB) (Xanthium sp.), yellow nutsedge (YNS) (Cypeus esculentus). Ample seeds are planted to give about 20 to 40 seedlings per row, after emergence, depending upon the size of the plants.

Using an analytical balance, 37.5 milligrams (mg) of the compound to be tested are weighed out on a piece of glassine weighing paper. The paper and compound are placed in a 60 milliliter (ml) wide-mouth clear bottle and dissolved in 45 ml of acetone or substituted solvent. Eighteen ml of this solution are transferred to a 60 ml wide-mouth clear bottle and diluted with 22 ml of a water and acetone mixture (19:1) containing enough polyoxyethylene sorbitan monolaurate emulsifier to give a final solution of 0.5% (v/v). The solution is then sprayed on a seeded flat on a linear spray table calibrated to deliver 40 gallons per acre (748 L/ha). The application rate is 0.25 Kg/ha.

After treatment, the flats are placed in the greenhouse at a temperature of 70 to 80°F and watered by sprinkling. Two weeks after treatment, the degree of injury or control is determined by comparison with untreated check plants of the same age. The injury rating from 0 to 100% is recorded for each species as percent control with 0% representing no injury and 100% representing complete control.

The results of the test are shown in the following Table 1.

TABLE 1

| Preemergence Herbicidal Activity Application Rate - 0.25 Kg/ha | | | | | | | | |
|---|---|---|---|---|---|---|---|---|
| Compd. No. | FT | GFT | WG | BSG | AMG | VL | CB | YNS |
| 1 | 100 | 100 | 100 | 100 | 100 | 100 | 100 | 95 |

Postemeraence Multi-weed Herbicide Test: This test is conducted in an identical manner to the testing procedure for the pre-emergence multi-weed herbicide test, except the seeds of the twelve different weed species are planted 10-12 days before treatment. Also, watering of the treated flats is confined to the soil surface and not to the foliage of the sprouted plants.

The results of the postemergence multi-weed herbicide test are reported in Table 2.

TABLE 2

| Postemergence Multi-weed Herbicidal Activity Application Rate - 0.25 Kg/ha | | | | | | | | |
|---|---|---|---|---|---|---|---|---|
| Compd. No. | FT | GFT | WG | BSG | AMG | VL | CB | YNS |
| 1 | 100 | 98 | 100 | 95 | 90 | 90 | 95 | 85 |

The compound of the present invention and its salts are useful as herbicides and can be applied in a variety of ways at various concentrations. In practice, the compounds are formulated into herbicidal compositions, by admixture, in herbicidally effective amounts, with the adjuvants and carriers normally employed for facilitating the dispersion of active ingredients for agricultural applications, recognizing the fact that the formulation and mode of application of a toxicant may affect the activity of the materials in a given application. Thus, these active herbicidal compounds or salts can be formulated as granules of relatively large particle size, as wettable powders, as emulsifiable concentrates, as powdery dusts, as flowables, as solutions or as any of several other known types of formulations, depending upon the desired mode of application. These formulations may contain as little as about 0.5% to as much as about 95% or more by weight of active ingredient. An herbicidally effective amount depends upon the nature of the seeds or plants to be controlled and the rate of application varies from about 0.01 to approximately 10 pounds per acre, preferably from about 0.02 to about 4 pounds per acre.

Wettable powders are in the form of finely divided particles which disperse readily in water or other dispersants. The wettable powder is ultimately applied to the soil either as a dry dust or as a dispersion in water or other liquid. Typical carriers for wettable powders include fuller's earth, kaolin clays, silicas and other readily wettable organic or inorganic diluents. Wettable powders normally are prepared to contain about 5% to about 95% of the active ingredient and usually also contain a small amount of wetting, dispersing, or emulsifying agent to facilitate wetting and dispersion.

Emulsifiable concentrates are homogeneous liquid compositions which are dispersible in water or other dispersant, and may consist entirely of the active compound or salt with a liquid or solid emulsifying agent, or may also contain a liquid carrier, such as xylene, heavy aromatic naphtha, isophorone and other non-volatile organic solvents. For herbicidal application, these concentrates are dispersed in water or other liquid carrier and normally applied as a spray to the area to be treated. The percentage by weight of the essential active ingredient may vary according to the manner in which the composition is to be applied, but in general comprises about 0.5% to 95% of active ingredient by weight of the herbicidal composition.

Granular formulations wherein the toxicant is carried on relatively coarse particles, are usually applied without dilution to the area in which suppression of vegetation is desired. Typical carriers for granular formulations include sand, fuller's earth, attapulgite clay, bentonite clays, montmorillonite clay, vermiculite, perlite and other organic or inorganic materials which absorb or which may be coated with the toxicant. Granular formulations normally are prepared to contain from about 1% to about 25% of active ingredients which may include surface-active agents such as heavy aromatic naphtha, kerosene or other petroleum fractions, or vegetable oils; and/or stickers such as destrins, glue or synthetic resins.

Typical wetting, dispersing or emulsifying agents used in agricultural formulations include, for example, the alkyl and alkylaryl sulfonates and sulfates and their salts; polyhydric alcohols; polyethoxylated alcohols; esters and fatty amines; and other types of surface-active agents, many of which are available in commerce. The surface-active agent, when used, normally comprises from 0.1% to 15% by weight of the herbicidal composition.

Dusts, which are free-flowing admixtures of the active ingredients with finely divided solids such as talc, clays, flours and other organic and inorganic solids which act as dispersants and carriers for the toxicant, are useful formulations for soil-incorporating application.

Pastes, which are homogeneous suspensions of a finely divided solid toxicant in a liquid carrier such as water or oil, are employed for specific purposes. These formulations normally contain about 5% to about 95% of active ingredient by weight, and may also contain small amounts of a wetting, dispersing or emulsifying agent to facilitate dispersion. For application, the pastes are normally diluted and applied as a spray to the area to be affected.

Other useful formulations for herbicidal applications include simple solutions of the active ingredient in a dispersant in which it is completely soluble at the desired concentration, such as acetone, alkylated naphthalenes, xylene and other organic solvents. Pressurized sprays, typically aerosols, wherein the active ingredient is dispersed in finelydivided form as a result of vaporization of a low boiling dispersant solvent carrier, such as the Freons, may also be used.

The phytotoxic compositions of this invention can be applied to the plants in any conventional manner. Thus, the dust and liquid compositions can be applied to the plant by the use of power dusters, boom and hand sprayers and spray dusters. The compositions can also be applied from airplanes as a dust or a spray or by rope wick applications because they are effective in very low dosages. In order to modify or control growth of germinating seeds or emerging seedlings, as a typical example, the dust and liquid compositions can be applied to the soil according to conventional methods and can be distributed in the soil to a depth of at least 1/2 inch below the soil surface. It is not necessary that the phytotoxic compositions be mechanically admixed with the soil particles since these compositions can also be applied merely by spraying or sprinkling the surface of the soil. The phytotoxic compositions of this invention can also be applied by addition to irrigation water supplied to the field to be treated. This method of application permits the penetration of the compositions into the soil as the water is absorbed therein. Dust compositions, granular compositions or liquid formulations applied to the surface of the soil can be distributed below the surface of the soil by conventional means such as discing, dragging, or mixing operations. In the following examples the herbicidal compound can be substituted with the herbicidal salt of the compound.

| EMULSIFIABLE CONCENTRATE FORMULATIONS | | | |
|---|---|---|---|
| General Formula with Ranges | | Specific Formula | |
| herbicidal compound | 5-55 | herbicidal compound | 24 |
| surfactant(s) | 5-25 | proprietary blend of oil-soluble sulfonates and polyoxyethylene ethers | 10 |
| | | polar solvent | 27 |
| solvent(s) | 20-90 | petroleum hydrocarbon | 39 |
| | 100% | | 100% |

| WETTABLE POWDER FORMULATIONS | | | |
|---|---|---|---|
| herbicidal compound | 3-90 | herbicidal compound | 80 |
| wetting agent | 0.5-2 | sodium dialkyl naphthalene sulfonate | 0.5 |

(continued)

| WETTABLE POWDER FORMULATIONS | | | |
|---|---|---|---|
| dispersing agent | 1-8 | sodium lignosulfonate | 7 |
| diluent(s) | 8.5-85.5 | attapulgite clay | 12.5 |
| | 100% | | 100% |

| EXTRUDED GRANULAR FORMULATIONS | | | |
|---|---|---|---|
| herbicidal compound | 1-20 | herbicidal compound | 10 |
| binding agent | 0-10 | lignin sulfonate | 5 |
| diluent(s) | 70-99 | calcium carbonate | 85 |
| | 100% | | 100% |

| FLOWABLE FORMULATIONS | | | |
|---|---|---|---|
| herbicidal compound | 20-70 | herbicidal compound | 45 |
| surfactant(s) | 1-10 | polyoxyethylene ether | 5 |
| suspending agent(s) | 0.05-1 | attagel | 0.05 |
| antifreeze agent | 1-10 | propylene glycol | 10 |
| antimicrobial agent | 1-10 | 1,2-benzisothiazoline-3-one | 0.03 |
| anti-foam agent | 0.1-1 | silicone defoamer | 0.02 |
| solvent | 7.95-76.85 | water | 39.9 |
| | 100% | | 100% |

When salts are used as the active ingredient in the herbicidal compositions of this invention it is recommended to use salts that are agriculturally acceptable.

The phytotoxic compositions of this invention can also contain other additives, for example, fertilizers, other herbicides and other pesticides, used as adjuvant or in combination with any of the above-described adjuvants. Fertilizers useful in combination with the active ingredients include, for example, ammonium nitrate, urea and superphosphate.

The herbicidal compounds of this invention can be used in combination with other herbicidal compounds for broader spectrum control of undesirable vegetation. Examples of other herbicidal compounds are as follows:

1. ANILIDES

Acetochlor - 2-chloro-N(ethoxymethyl)-6'-ethyl-o-acetotoluidide (2-chloro-N-(ethoxymethyl)-N-(2-ethyl-6-methylphenyl)acetamide)
Alachlor - 2-chloro-2',6'-diethyl-N-(methoxymethyl) acetanilide
Metolachlor - 2-chloro-N-(2-ethyl-6-methylphenyl)-N-(2-methoxy-1-methylethyl)acetamide
Propanil - N-(3,4-dichlorophenyl)propionanilide

2. TRIAZINES

Atrazine - 2-chloro-4-(ethylamino)-6-isopropylamino)-s-triazine
Cyanazine - 2-chloro-4-(1-cyano-1-methylethylamino)-6-ethylamino-s- triazine
Metribuzin - 4-amino-6 -tert-butyl-3-(methylthio)-1,2,4-triazin-5(4H)-one

3. THIOCARBAMATES

Molinate - S-ethyl hexahydro-1H-azepine-1-carbothioate
Butylate - S-ethyl diisobutylthiocarbamate

4. UREAS

Monuron - 3-(p-chlorophenyl)-1,1-dimethylurea
Linuron - 3-(3,4-dichlorophenyl)-1-methoxy-1-methylurea

5. TOLUIDINES

Trifluralin - α,α,α-trifluoro-2,6-dinitro-N,N-dipropyl-p-toluidine
Pendimethalin - N-(1-ethylpropyl)-3,4-dimethyl-2,6-dinitrobenzeneamine

6. HORMONES

2,4-D - (2,4-dichlorophnoexy) acetic acid
MCPA - (2-methyl-4-chlorophenoxy) acetic acid

7. DIAZINES

Bentazon - 3-isopropyl-1H-2,3,1-benzothiadiazin-4(3H)-one 2,2-dioxide
Oxadiazon - 2-tert-butyl-4-(2,4-dichloro-5-isopropoxyphenyl)-$\Delta^2$-1,3,4-oxadiazolin-5-one

8. DIPHENYL ETHERS

Acifluorfen - sodium 5-[2-chloro-4-(trifluoromethyl)phenoxy]-2-nitrobenzoate
Fluazifop-butyl -(±)-butyl 2-[4[(5-(trifluoromethyl)-2-pyridinyl)oxy]phenoxy]propanoate
Chlomethoxynil - 2,4-dichlorophenyl 3-methoxy-4-nitrophenyl ether

9. IMIDAZOLINONES
Imazaquin - 2-[4,5-dihydro-4-methyl-4-(1-methylethyl)-5-oxo-1H-imidazol-2-yl]-3-quinolin carboxylic acid
10. SULFONYL UREAS

Bensulfuron methyl - methyl 2-[[[[[(4,6-dimethoxypyrimidin-2-yl)amino]carbonyl]amino]sulfonyl]methyl]benzoate
Chlorimuron ethyl - ethyl 2-(((((4-chloro-6-methoxypyrimidin-2-yl)amino)carbonyl)amino)sulfonyl) benzoate

11. MISCELLANEOUS COMPOUNDS

Dimethazone - 2-(2-chlorophenyl)methyl-4,4-dimethyl-3-isoxazolidinone
Norflurazon - 4-chloro-5-(methylamino)-2-α,α,α-trifluoro-m-tolyl)-3-(2H)-pyridazinone
Dalapon - 2,2-dichloropropionic acid
Glyphosate - isopropyl amine salt of N-(phosphonomethyl) glycine
Fenoxaprop-ethyl - (+)-ethyl-2,4-((6-chloro-2-benzoxazoly loxy)phenoxy)propanoate

## Claims

1. 2-(2-chloro-3-ethoxy-4-ethylsulfonyl benzoyl)-5-methyl-1,3-cyclohexanedione and its salts.

2. A method of controlling undesirable vegetation comprising applying to the area where control is desired an herbicidally effective amount of 2-(2-chloro-3-ethoxy-4-ethylsulfonyl benzoyl)-5-methyl-1,3-cyclohexanedione or its salts.

3. An herbicidal composition comprising 2-(2-chloro-3-ethoxy-4-ethylsulfonyl benzoyl)-5-methyl-1,3-cyclohexanedione or its salts and an inert carrier thereof.

## Patentansprüche

1. 2-(2-Chlor-3-ethoxy-4-ethylsulfonylbenzoyl)-5-methyl-1,3-cyclohexandion und seine Salze.

2. Verfahren zur Bekämpfung unerwünschter Vegetation, bei dem auf die Fläche, auf der die Bekämpfung erwünscht

ist, eine herbicid wirksame Menge 2-(2-Chlor-3-ethoxy-4-ethylsulfonylbenzoyl)-5-methyl-1,3-cyclohexandion oder eines Salzes davon aufgebracht wird.

3. Herbicide Zusammensetzung, die 2-(2-Chlor-3-ethoxy-4-ethylsulfonylbenzoyl)-5-methyl-1,3-cyclohexandion oder dessen Salze und einen inerten Trägerstoff dafür enthält.

**Revendications**

1. 2-(2-chloro-3-éthoxy-4-éthylsulfonylbenzoyl)-5-méthyl-1,3-cyclohexanedione et ses sels.

2. Procédé pour lutter contre de la végétation indésirable, comprenant l'application à la zone où cette lutte est désirée une quantité efficace du point de vue herbicide de 2-(2-chloro-3-éthoxy-4-éthylsulfonylbenzoyl)-5-méthyl-1,3-cyclohexanedione ou d'un des sels.

3. Composition herbicide comprenant de la 2-(2-chloro-3-éthoxy-4-éthylsulfonylbenzoyl)-5-méthyl-1,3-cyclohexanedione ou ses sels et un support inerte.